# EUROPEAN PATENT APPLICATION

(11) **EP 2 389 878 A1**
(43) Date of publication of application: **30.11.2011**
(21) Application number: 11250568.0
(22) Date of filing: 27.05.2011
(51) Int. Cl.: A61B 17/122

(54) **Surgical hemostatic clip including work-hardened, movement-inhibiting structure**

(30) Priority: 28.05.2010 US 789967
(71) Applicant: Tyco Healthcare Group LP, New Haven, CT 06511 (US)
(72) Inventor: Whitfield, Kenneth H., North Haven, CT 06473 (US)
(74) Representative: Soames, Candida Jane

(57) **Abstract**

A surgical clip includes a clip body defining opposed, first and second leg portions. Each of the first and second leg portions includes a distal end region and a proximal end region, with the proximal end regions connected at an apex to form a bail portion. The surgical clip also includes a work-hardened, movement-inhibiting structure defined in the clip body adapted to enhance clip retention on tissue.

## Description

### BACKGROUND

### 1. Technical Field

The present disclosure relates to hemostatic surgical clips for application to blood vessels or body tissue and, more particularly, to hemostatic surgical clips including a work-hardened, movement-inhibiting structure and a method of manufacturing the same.

### 2. Discussion of Related Art

Ligation or occlusion of veins, arteries or blood vessels is a necessary part of some surgical procedures. Typically, a blood vessel to be severed requires closure on both sides of a severance site before actual cutting takes place. In the past, surgeons used thread or suture material to tie a blood vessel prior to severing the vessel.

The procedure of tying a blood vessel using thread or suture material was often time-consuming and requires dexterity on the part of the surgeon to properly close the vessel. In many instances, the assistance of a nurse or attending surgeon was necessary to perform this procedure to perfect grasping and tying the vessel, then repeatedly testing the vessel to ensure closure. If complete closure of the vessel was not achieved using the suture material, then the sequence was repeated.

Surgical clips and hemostatic surgical clip appliers greatly enhance the art of vessel occlusion. Surgical clips are now commonly used for vessel ligation and occlusion. Examples of surgical hemostatic clips are described in U.S. Pat. No. 5,713,911 to Racenet et al.; U.S. Pat. No. 5,626,592 to Phillips et al.; U.S. Pat. No. 5,501,693 to Gravener; U.S. Pat. No. 5,201,746 to Shichman; and U.S. patent application Ser. No. 11/338,911 filed on Jan. 23, 2006, the disclosures of which are incorporated herein by reference in their entireties.

Many factors impact upon the design of a surgical hemostatic clip to achieve proper tissue exudation and occlusion. The clip should not slip or become dislodged from a blood vessel after it has been applied. If the clip is not securely positioned, blood or other bodily fluid may begin flowing into the surgical site through the unclamped vessel. In such case, the surgery may be delayed while the surgeon locates and reclamps the vessel. Depending upon the type and location of the surgery, reclamping the vessel may be difficult, and reduce an overall productivity of the procedure. A clip should fully and completely close about a vein, artery, vessel or other conduit and completely stop the flow of blood or fluid therethrough. A clip that does not completely occlude blood or fluid flow may be unsuitable for many surgical applications and have to be removed or supplemented thus requiring application of a second clip.

Surgical hemostatic clips are generally U-shaped or V-shaped in configuration and define a pair of legs joined at one end by an apex or crown and spaced apart at opposing ends to define an opening therebetween. Clips often have a bail portion, which is the arcuate or V-shaped proximal portion, and substantially parallel legs extending from the bail portion. The inside surfaces of the clip legs may be constructed in a manner to improve the occluding functions of the clip as well as to restrict longitudinal and transverse dislocation of the clip after it has been applied to the target blood vessel or other conduit.

A surgical clip should be simple to manipulate and handle and preferably should be simple and inexpensive to manufacture. Surgical clips are generally made of biocompatible, metallic or non-metallic materials, e.g., polymeric materials, or bioabsorbable materials. Surgical clips are often formed of tantalum or stainless steel which are capable of being deformed and possess sufficient strength to retain the deformation when clamped about a blood vessel.

The clip legs should not shift laterally with respect to each other during closure. Clip legs that have shifted a relatively small amount may be referred as "twisted". If the misalignment is relatively large, the clip may be referred to as having "scissored". Scissoring or twisting of a clip may result in damage to tissue and enlargement of the gap between the clip legs.

Another factor in the design of surgical hemostatic clips relates to its storage and advancement through a clip applying instrument designed to apply multiple clips. In certain commercially-available clip appliers, the surgical clips are stored in a linear array extending longitudinally along the instrument with the legs of one clip contacting the bail portion of the preceding clip. As each clip is applied, an advancing force is applied to the last clip in the row and each clip pushes or advances the clip in front of it such that the distal-most clip is positioned within the jaws or other suitable position. After the jaws receive a clip, the jaws are brought together to close the clip around a tissue structure. The shape, size and overall geometry of a surgical clip may affect its movement through the clip applying apparatus.

A need exists for an improved surgical hemostatic clip to provide optimum vessel occlusion and optimal clip retention on tissue during a surgical procedure.

### SUMMARY

The present disclosure relates to a surgical clip including a clip body defining opposed, first and second leg portions. Each of the first and second leg portions includes a distal end region and a proximal end region, with the proximal end regions connected at an apex to form a bail portion. The surgical clip also includes a work-hardened, movement-inhibiting structure defined in the clip body adapted to enhance clip retention on tissue.

The present disclosure relates to a surgical clip including a clip body defining opposed, first and second leg portions. Each of the first and second leg portions includes a distal end region and a proximal end region, with the proximal end regions connected at an apex to form a bail portion. Each of the first and second leg portions further includes an inner surface defining a tissue-contacting surface between which tissue is clamped during application of the surgical clip thereto. The surgical clip also includes a work-hardened, movement-inhibiting structure defined in at least a portion of the tissue-contacting surface adapted to enhance clip retention on tissue.

The present disclosure also relates to a method of manufacturing a work-hardened, movement-inhibiting structure of a surgical clip. The method includes the initial step of providing a surgical clip including a generally U-shaped clip body defining opposed, first and second leg portions. Each of the first and second leg portions includes a proximal end region. The proximal end regions are connected at an apex to form a bail portion. The method includes the steps of providing a fixture configured to supportively secure the surgical clip to allow for application of a pressing force to a first surface of the bail portion of the clip body, and releasablely coupling the surgical clip to the fixture. The method also includes the steps of applying a predetermined pressing force to the first surface of the bail portion to form an elongated recess therein, such that the elongated recess has a generally C-shaped cross section, thereby forming a work-hardened, movement-inhibiting structure of the surgical clip, and removing the surgical clip from the fixture.

### BRIEF DESCRIPTION OF THE DRAWINGS

Features of the presently disclosed hemostatic surgical clips including a work-hardened, movement-inhibiting structure and a method of manufacturing the same will become apparent to those of ordinary skill in the art when descriptions of various embodiments thereof are read with reference to the accompanying drawings, of which:

FIG. 1 is right side perspective view of an embodiment of a surgical clip including a work-hardened, movement-inhibiting structure in accordance with the present disclosure;

FIG. 2 is a left side perspective view of the surgical clip of FIG. 1;

FIG. 3 is a top front perspective view of the surgical clip of FIG. 1;

FIG. 4 is a cross-sectional view of the first leg portion of the surgical clip of FIG. 1 taken along section lines 4-4 showing a work-hardened, movement-inhibiting structure defined in the tissue-contacting surface thereof;

FIG. 5 is a cross-sectional view of the second leg portion of the surgical clip of FIG. 1 taken along section line 5-5 showing an alignment rib defined on the tissue-contacting surface thereof;

FIG. 6 is perspective view illustrating the application the surgical clip of FIG. 1 to a tubular organic structure;

FIG. 7 is right side perspective view of an embodiment of a surgical clip including two, work-hardened, movement-inhibiting structures in accordance with the present disclosure;

FIG. 8 is a left side perspective view of the surgical clip of FIG. 7;

FIG. 9 is a perspective view illustrating the application the surgical clip of FIG. 7 to a tubular organic structure;

FIG. 10 is a cross-sectional view of the first leg portion of the surgical clip of FIG. 7 taken along section line 10-10 showing a work-hardened, movement-inhibiting structure defined in the tissue-contacting surface thereof and a work-hardened, movement-inhibiting structure defined in the force-receiving surface thereof;

FIG. 11 is a cross-sectional view of the second leg portion of the surgical clip of FIG.8 taken along section line 11-11 showing the work-hardened, movement-inhibiting structure defined in the force-receiving surface thereof and an alignment rib projecting from the tissue-contacting surface thereof;

FIG. 12 is right side perspective view of another embodiment of a surgical clip including a work-hardened, movement-inhibiting structure in accordance with the present disclosure;

FIG. 13 is a top front perspective view of the surgical clip of FIG. 12; and

FIG. 14 is a flowchart illustrating a method of manufacturing a work-hardened, movement-inhibiting structure of a surgical clip according to an embodiment of the present disclosure.

### DETAILED DESCRIPTION

Hereinafter, embodiments of the presently disclosed hemostatic surgical clips including a work-hardened, movement-inhibiting structure and a method of manufacturing the same are described with reference to the accompanying drawings. Like reference numerals may refer to similar or identical elements throughout the description of the figures. As shown in the drawings and as used in this description, and as is traditional when referring to relative positioning on an object, the term "proximal" refers to that portion of the clip, or structure thereof, that is closer to the user and the term "distal" refers to that portion of the clip, or structure thereof, that is farther from the user.

This description may use the phrases "in an embodiment," "in embodiments," "in some embodiments," or "in other embodiments," which may each refer to one or more of the same or different embodiments in accordance with the present disclosure. For the purposes of the description, a phrase in the form "A and/or B" means "(A), (B), or (A and B)".

As it is used in this description, "metal" generally refers any metal or any combination of one or more metals or alloys thereof. As it is used in this description, "cold working" generally refers to work hardening that occurs in metalworking processes that induce plastic deformation to cause a shape change and affect the metal clip material properties. For example, yield strength is increased in a cold-worked material. For the purposes herein, the term "cold working" is interchangeable with the term "cold forming". For the purposes herein, the term "work hardening" is interchangeable with the term "strain hardening".

Various embodiments of the present disclosure provide hemostatic surgical clips including a work-hardened, movement-inhibiting structure suitable for application to veins, arteries, blood vessels, or other body tissues to achieve ligation or occlusion. The presently disclosed hemostatic surgical clip embodiments including a work-hardened, movement-inhibiting structure have improved folding characteristics to enhance clip retention on tissue.

Although the following description describes hemostatic surgical clips with improved folding characteristics suitable for application to tissue to achieve complete ligation or occlusion, the teachings of the present disclosure may also apply to surgical clips used to achieve partial ligation or occlusion. The teachings of the present disclosure may also apply to wound or incision closure devices, or other devices, e.g., surgical fasteners for implantable devices.

Various embodiments of the presently disclosed surgical hemostatic clip including a work-hardened, movement-inhibiting structure are designed to be applied to body tissue by a surgical clip applying apparatus. A surgical clip applying apparatus generally has a pair of jaws to position the clip relative to tissue and to deform or close the clip about tissue, usually by bending the clip near its apex such that the legs of the clip close about the tissue, such as, for example, a blood vessel. Examples of surgical instruments that may be suitable for use in the application of surgical hemostatic clip embodiments described herein are set forth in commonly assigned U.S. Pat. No. 4,509,518 to McGarry et al.; U.S. Pat. Nos. 5,084,057 and 5,100,420 to Green et al.; U.S. Pat. No. 5,269,792 to Kovac; and U.S. patent application Ser. No. 11/245,523 filed on Oct. 7, 2005, the disclosures of which are incorporated herein by reference in their entireties.

Cold working, also known as cold forming or cold forging, involves the plastic deformation of a metal at a temperature below its recrystallization point. Cold forming processes are usually, but not necessarily, conducted at the ambient temperature. During cold working, it may take a significant amount of energy to affect a shape change. Some of the mechanical energy expended during a plastic deformation may appear in the form of heat, while some amount of the energy may be stored in the material. This stored energy may be associated with defects called dislocations created by fluctuations in local stress fields within the material during the plastic deformation. Work hardening, also known as strain hardening, may be defined as the increase in the yield stress of the metal after it has been deformed. This increase in the yield stress of the metal generally makes it more difficult to further deform the material. Furthermore, the degree of work hardening for a given plastic deformation depends on the initial hardness of the metal. Thus annealed metal may be hardened to ¼ hard for a given deformation while ¼ hard material may be hardened to ¾ hard for the same deformation. Embodiments of the presently disclosed hemostatic surgical clip include a movement-inhibiting structure formed by cold working to enhance clip retention on tissue.

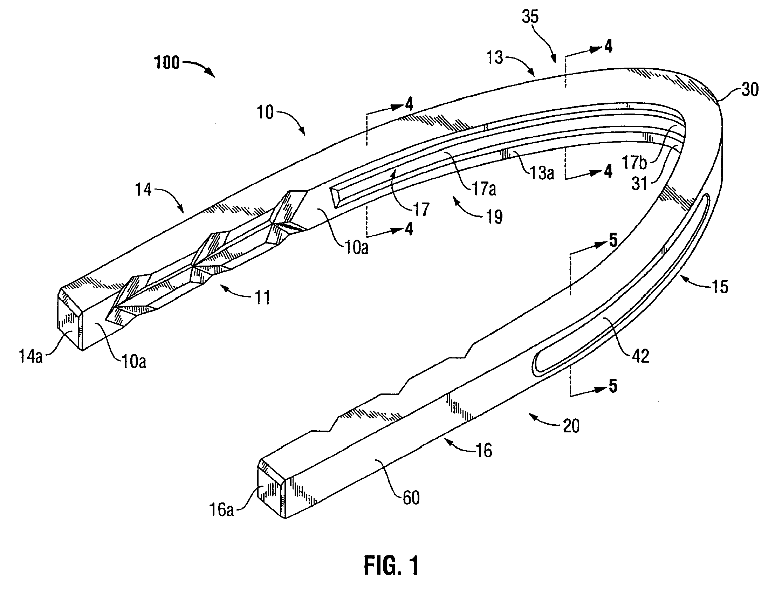

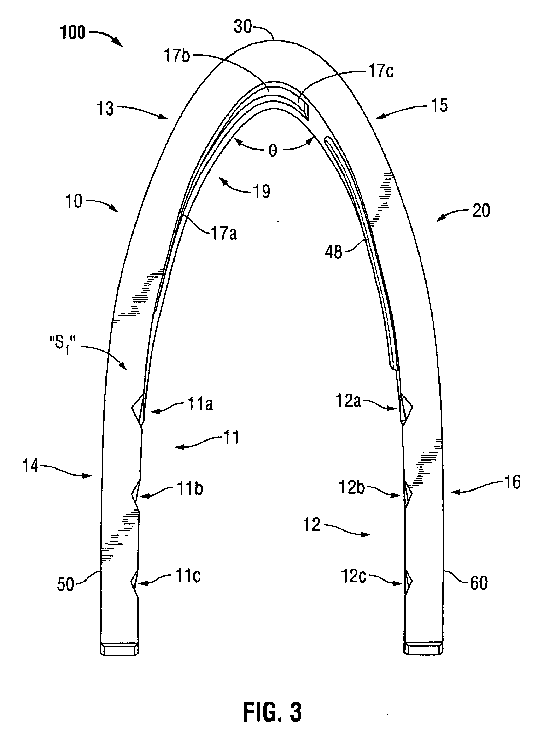

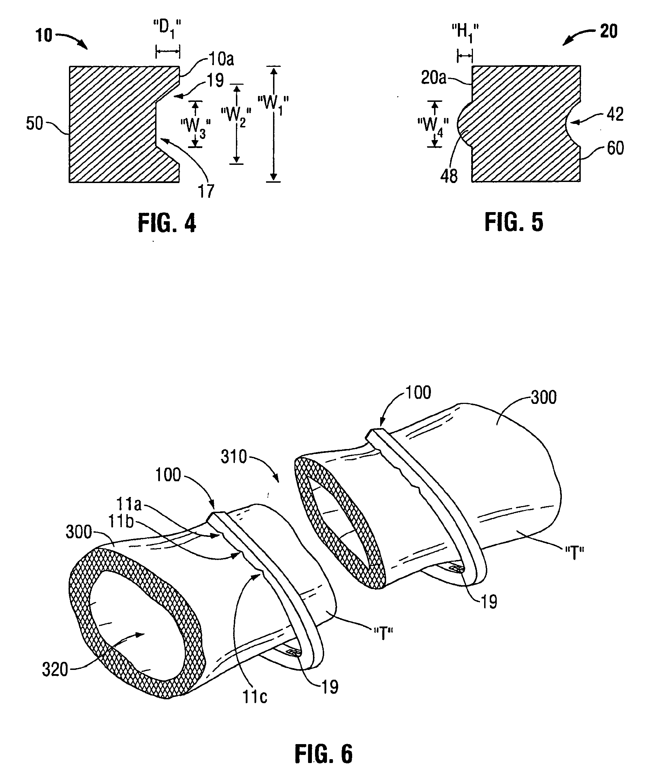

A surgical hemostatic clip 100 according to an embodiment of the present disclosure is shown in FIGS. 1 through 3 and includes opposed, first and second leg portions 10 and 20. In embodiments, the width "W₁" (shown in FIG. 4) of the first and second leg portions 10 and 20 is in the range of about 0.034 inches to about 0.036 inches. First and second leg portions 10 and 20 include distal end regions 14 and 16, respectively, and proximal end regions 13 and 15, respectively. Proximal end regions 13, 15 are curved and join at an angle θ (shown in FIG. 3) to define an apex 30 and together form a generally V-shaped or U-shaped bail portion 35 of the clip. Distal end regions 14 and 16 terminate in free ends 14a and 16a, respectively, and may be rectilinear in shape and initially parallel to one another. The shape and size of the distal end regions 14, 16, the proximal end regions 13, 15 and the apex 30, which are described in more detail below, may be varied from the configuration depicted in FIGS. 1 through 3.

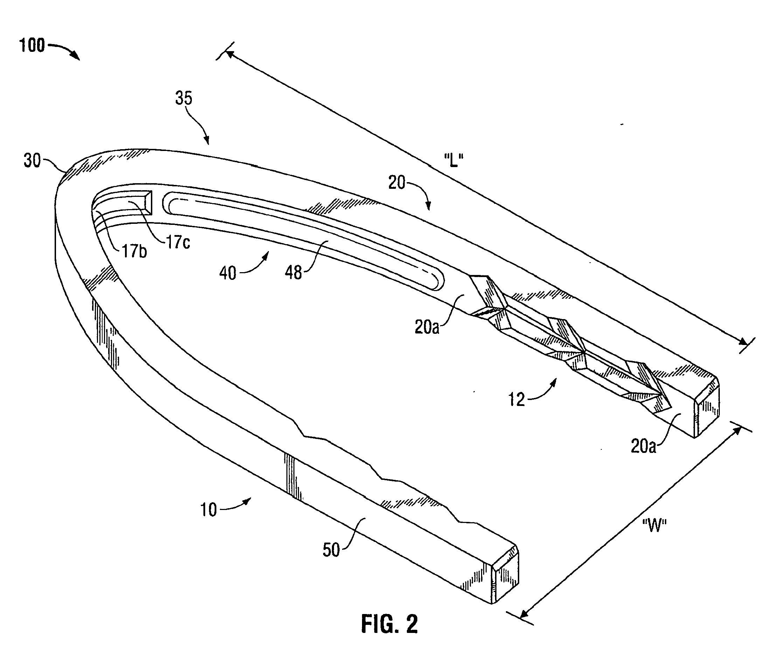

Surgical hemostatic clip 100 may be of any dimensions suitable for application to body tissue. Referring to FIG. 2, the clip 100 has an overall length "L" defined by the distance between the distal ends 14a and 16a of the first and second leg portions 10 and 20, respectively, and the outer edge of the apex 30. Clip 100 has a width "W" defined by the distance between the outer surfaces of the first and second leg portions 10 and 20. In one embodiment, the length "L" of the clip is about 0.3 inches and the width "W" of the clip is from about 0.180 to about 0.224 inches. Clip 100 may be dimensioned such that the ratio of the width "W" to the overall length "L" (i.e., the ratio W/L) ranges from about 0.54 to about 0.68. First and second leg portions 10 and 20 are preferably, but not necessarily, of equal length.

Apex 30 is configured to facilitate the crimping of the clip 100 when the clip is applied to body tissue by a clip applicator. In embodiments, the angle θ formed by the proximal end regions 13, 15 is in the range of about 129° to about 131°. In embodiments, the apex 30 is characterized by an angle θ of less than about 100°. In one embodiment, the apex 30 has a radius of curvature of about four percent (4%) of the length "L" of the clip 100. As one of ordinary skill in the art will readily recognize, other clip embodiments may be provided with different dimensions and angles.

The inner surfaces of the first leg portion 10 and the second leg portion 20 of the hemostatic clip 100 define tissue-contacting surfaces 10a and 20a, respectively, between which tissue is clamped during application of the surgical hemostatic clip 100 thereto. The outer surfaces of the first and second leg portions 10 and 20 define force-receiving surfaces 50 and 60, respectively, for receiving the compression force from the jaws of the clip applying apparatus.

A work-hardened, movement-inhibiting structure 19 adapted to enhance clip retention on tissue is defined in the tissue-contacting surfaces 10a and 20a. In embodiments, the work-hardened, movement-inhibiting structure 19 may be disposed entirely within the bail portion 35 of the clip 100. Movement-inhibiting structure 19 may also include end portions thereof defined in the distal portion 14 of the first leg portion 10 and/or the distal portion 16 of the second leg portion 20. The presently disclosed surgical clip 100 including a work-hardened, movement-inhibiting structure 19 in the closed position is such that it is relatively immovable if inadvertently hit by an instrument or sponge or the like.

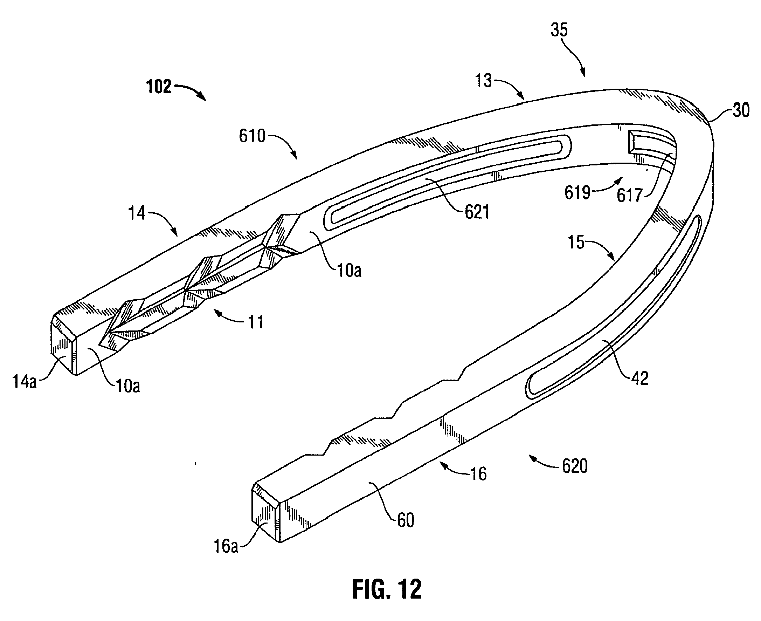

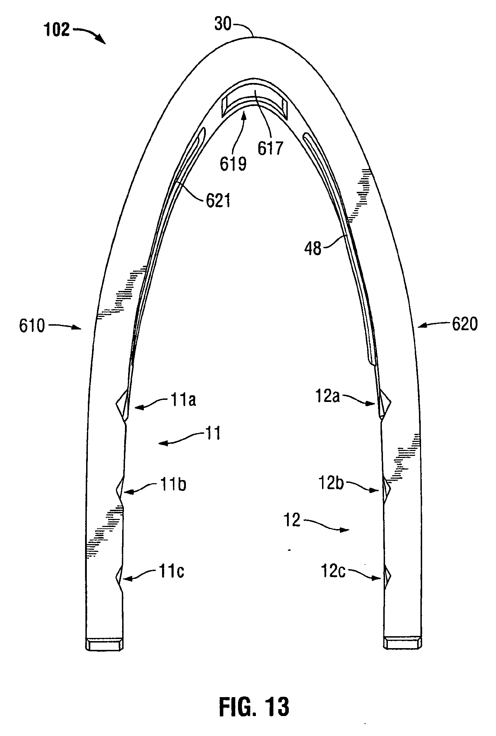

As shown in FIG. 3 and cooperatively shown in FIGS. 1 and 2, the work-hardened, movement-inhibiting structure 19 is an elongated recess or depression 17 including a first portion 17a formed in the tissue-contacting surface 10a disposed along the proximal end region 13 of the first leg portion 10, a second portion 17b formed in the inner surface 31 of the apex 30, and a third portion 17c formed in the tissue-contacting surface 20a disposed along a portion of the proximal region 15 of the second leg portion 20. In other embodiments, the presently disclosed work-hardened, movement-inhibiting structure may be configured as a recess of relatively short length (e.g., 617 shown in FIGS. 12 and 13) formed in the inner surface 31 of the apex 30, or portion thereof.

Movement-inhibiting structure 19 is formed by cold working the clip 100. For example, the movement-inhibiting structure 19 may be formed in the clip 100 by fixturing the clip and applying an appropriate stamping force to the inner surfaces 13a, 31 and 15a of the proximal end region 13, apex 30 and proximal end region 15, respectively, to form the recess 17 along the apex 30 and portions of the tissue-contacting surfaces 10a and 20a. Corresponding protuberances (not shown) may appear on the force-receiving surfaces and/or the clip material may "swage out" along the periphery of the clip to a limited degree.

Referring to FIG. 4, the recess 17 has a generally C-shaped cross section. This C-shape is formed by stamping or coining the originally constant section of the clip to form a recess. One purpose of such a recess is to increase the second moment of area of the highly deformed apex area by redistributing the material of the initial area cross section to a cross section that increases the second moment of area in the work-hardened, movement-inhibiting structure 19. This has three distinct benefits. Firstly, the initial stamping or coining operation work hardens the area adjacent the recess, resulting in further work hardening when the apex 30 is subsequently deformed. Optionally, the hardening caused by the initial coining may be controllably removed by annealing. Secondly, increased second moment of area of the apex section creates additional work hardening when the apex 30 is deformed and thirdly, the increased second moment of area of the apex section better resists opening of the apex once deformed. While shown and discussed as a C-section, any section which increase the second moment of area from an initial wire form (typically a substantially rectangular section) will provide these benefits to the work-hardened, movement-inhibiting structure 19. For example an overall I-beam section or a section with a hexagonal recess will increase the second moment of area. Once deformed, any of these sections provide enhanced resistance to opening of the apex and the work-hardened, movement-inhibiting structure 19 of the clip. It is undesirable to remove material from the apex 30 while creating the recess 17 or other features which increases the second moment of area in the work-hardened, movement-inhibiting structure 19.

Recess 17 has a depth "D₁", a width "W₂" at a plane defined by the tissue-contacting surface 10a, and a width "W₃" at the base of the recess 17. In one embodiment, the depth "D₁" is in the range of about 0.005 to about 0.007 inches. In some embodiments, the depth "D₁" is in the range of about 0.001 to about 0.010 inches, the width "W₂" is in the range of about 0.020 to about 0.030 inches and the width "W₃" is in the range of 0.005 to about 0.016 inches.

As shown in FIG. 2, the tissue-contacting surface 20a includes a tissue-gripping structure 40 configured as an elongated alignment rib 48 projecting from the tissue-contacting surface 20a. Tissue-gripping structure 40 may be formed by machining the clip 100 and/or other metal or polymer processing techniques. For example, the clip 100 may be molded with the alignment rib 48 formed thereon. An elongated depression 42 (shown in FIGS. 1 and 5) may be formed in the force-receiving surface 60 upon formation of the alignment rib 48. In embodiments, the first portion 17a of the recess 17 is adapted to receive the alignment rib 48 upon movement of the first and second leg portions 10 and 20 from the open to closed position.

Alignment rib 48 has an approximately half-hemispherical cross section. Rib 48 has a height "H₁" and a width "W₄" at its base. In some embodiments, the height "H₁" is in the range of about 0.001 to about 0.006 inches and the width "W₄" is in the range of about 0.005 to about 0.030 inches. In embodiments, the width "W₃' at the base of the recess 17 may be substantially the same as the width "W₄" at the base of the rib 48, e.g., to permit tissue exudation during the application of the hemostatic clip 100 to tissue. The shape and size of the rib 48 may be varied from the configuration depicted in FIG. 5.

As cooperatively shown in FIGS. 1 and 2, the clip 100 may additionally include a tissue-gripping structure 11 formed in the tissue-contacting surface 10a disposed along a portion of the distal end region 14 of the first leg portion 10, and a tissue-gripping structure 12 formed in the tissue-contacting surface 20a disposed along a portion of the distal end region 16 of the second leg portion 20. Tissue-gripping structures 11 and 12 each includes a plurality of multi-faceted, movement-inhibiting structures defining a plurality of recesses disposed in a pattern on the opposed, tissue-contacting surfaces 10a and 20a. When the hemostatic clip 100 is compressed to a closed position, the plurality of recesses of the tissue-gripping structure 11 are positioned in juxtaposed alignment with the plurality of recesses of the tissue-gripping structure 12 forming a pattern of polygonal shapes that cooperatively inhibit dislocation of the hemostatic clip 100 with respect to the body tissue (e.g., "T" shown in FIG. 6) to which it is applied.

Tissue-gripping structures 11 and 12 define a plurality of generally V-shaped open areas or notches in the upper and/or lower surfaces of the first and second leg portions 10 and 20, which permit tissue exudation during the application of the hemostatic clip 100 to tissue. For example, the first and second leg portions 10 and 20 may each include three open areas (e.g., 11a-11c and 12a-12c, respectively, shown in FIG. 3) in the upper surface "S₁" of the clip 100 defined by a plurality of movement-inhibiting structures of the tissue-gripping structures 11 and 12. The exudation of tissue into the open areas 11a-11c and 12a-12c further inhibits movement of the hemostatic clip 100 relative to the vessel to which it is applied.

FIG. 6 illustrates the application of the presently disclosed hemostatic clip 100 including a work-hardened, movement-inhibiting structure to body tissue "T". As shown in FIG. 6, a tubular organic structure such as blood vessel 300 is clipped in two locations with clips 100 of the present disclosure, thereby closing interior passageway 320 of the blood vessel and permitting a division 310 of the blood vessel 300 by a knife blade slicing between the clips 100. Clips 100 seal the newly created ends of the blood vessel 300 such that the flow of blood therethrough is completely occluded. However, while the flow of blood through the vessel passageway 320 is stopped, the open areas 11a-11c and/or open areas 12a-12c (not explicitly shown in FIG. 6) formed by the intersection of the tissue-gripping structures 11 and 12 with the surface "S₁" (shown in FIG. 3) permit the flow of nourishing body fluid within the wall of the blood vessel 300 to the portion of the blood vessel tissue "T" located between the tissue-gripping structures 11 and 12. This advantageous feature reduces the possibility of tissue necrosis.

When the first and second leg portions 10 and 20 are moved from the open to closed position, the deformation of the bail portion 35 may increase the concentration of dislocations in the work-hardened, movement-inhibiting structure 19. As the dislocations accumulate, the dislocations may interact with one another and serve as pinning points or obstacles that significantly impede their motion, requiring a greater amount of force to be applied to overcome the barrier, thereby enhancing clip retention on tissue.

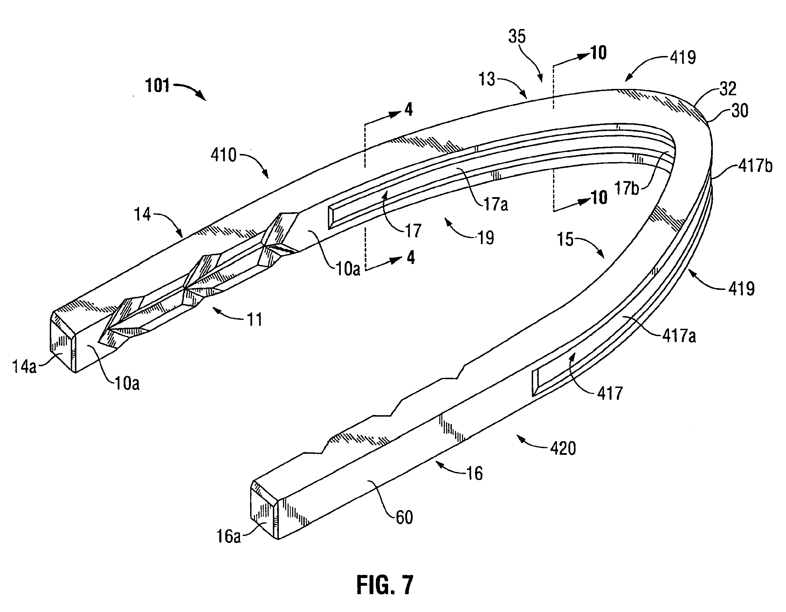

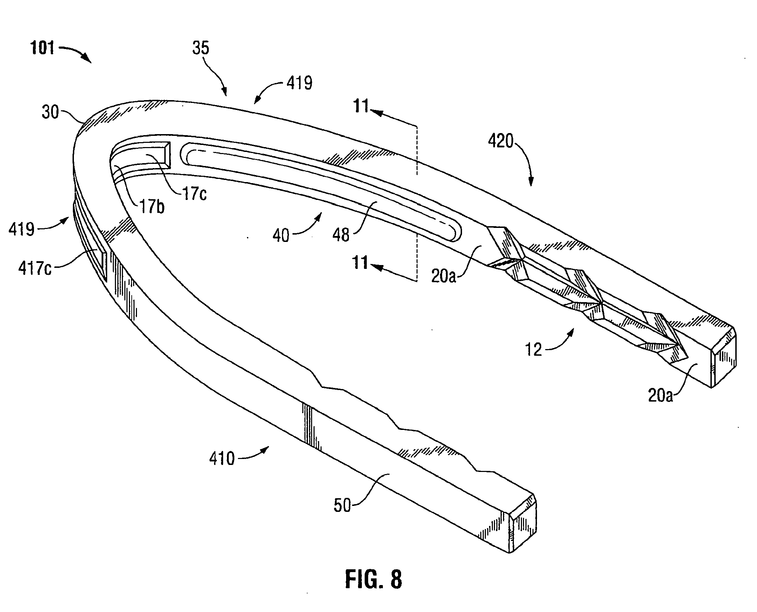

A surgical hemostatic clip 101 according to an embodiment of the present disclosure is shown in FIGS. 7 and 8 and includes opposed, first and second leg portions 410 and 420. Surgical hemostatic clip 101 is similar to the hemostatic clip 100 of FIGS. 1 through 3, except for the addition of a work-hardened, movement-inhibiting structure 419, which is defined in the force-receiving surfaces 50 and 60 of the first and second leg portions 410 and 420. First and second leg portions 410 and 420 are similar to the first and second leg portions 10 and 20 of the clip 100 shown in FIGS. 1 through 3 and, in the interests of brevity, not all features are discussed.

Surgical hemostatic clip 101 includes the work-hardened, movement-inhibiting structure 19 of FIGS. 1 through 3 and a work-hardened, movement-inhibiting structure 419. As cooperatively shown in FIGS. 7 and 8, the work-hardened, movement-inhibiting structure 419 is an elongated recess or depression 417 including a first portion 417a formed in the force-receiving surface 60 disposed along the proximal end region 16 of the second leg portion 420, a second portion 417b formed in the outer surface 32 of the apex 30, and a third portion 417c formed in the force-receiving surface 50 disposed along at least a portion of the proximal region 13 of the first leg portion 410, wherein the first, second and third portions 417a, 417b and 417c, respectively, are formed by cold working the clip 101. In some embodiments, the work-hardened, movement-inhibiting structure 419 may be disposed substantially entirely within the bail portion 35 of the clip 101. In other embodiments, the work-hardened, movement-inhibiting structure 419 may include end portions thereof defined in the distal portion 14 of the first leg portion 410 and/or the distal portion 16 of the second leg portion 420.

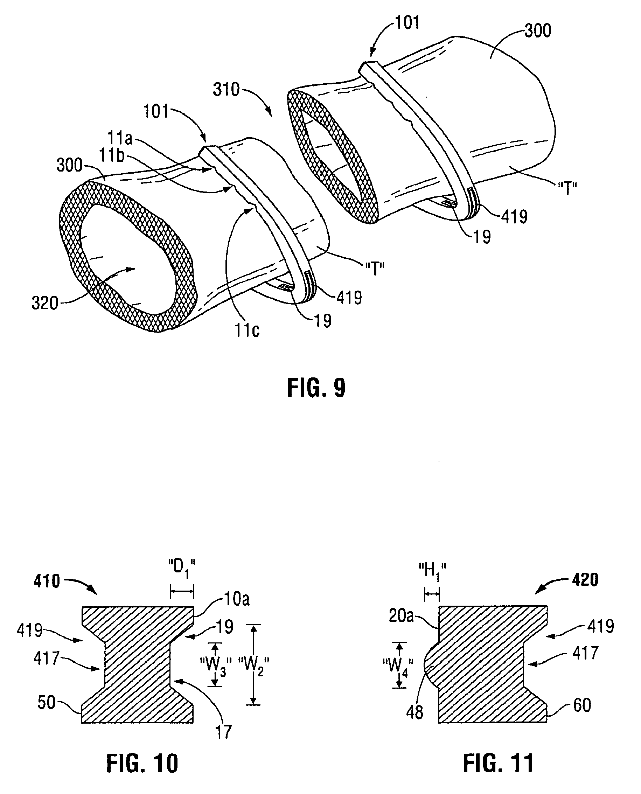

FIG. 9 illustrates the application of the presently disclosed hemostatic clip 101 to body tissue "T". As shown in FIG. 9, a tubular organic structure such as blood vessel 300 is clipped in two locations with clips 101, thereby closing interior passageway 320 of the blood vessel and permitting a division 310 of the blood vessel 300 by a cutting instrument slicing between the clips 101. Clips 101 include the work-hardened, movement-inhibiting structure 19 of FIGS. 1 through 6 defined in the tissue-contacting surfaces 10a and 20a, and the work-hardened, movement-inhibiting structure 419 of FIGS. 7 and 8 defined in the force-receiving surfaces 50 and 60, e.g., to enhance clip retention on tissue and minimize twist during the application of the clips to tissue.

FIGS. 10 and 11 are cross-sectional views of portions of the first and second leg portions 410 and 420, respectively. As shown in FIG. 10, the first leg portion 410 includes a recess 17 having a generally C-shaped cross section formed in the tissue-contacting surface 10a thereof, and a recess 417 having a generally C-shaped cross section formed in the force-receiving surface 50 thereof. Recess 17 has a depth "D₁", a width "W₂" at a plane defined by the tissue-contacting surface 10a, and a width "W₃" at the base of the recess 17. In one embodiment, the depth "D₁" is in the range of about 0.005 to about 0.007 inches. In some embodiments, the depth "D₁" is in the range of about 0.001 to about 0.010 inches, the width "W₂" is in the range of about 0.020 to about 0.030 inches, and the width "W₃" is in the range of about 0.005 to about 0.016 inches. Recess 417 formed in the force-receiving surface 50 of the first leg portion 410 may have the same dimensions as the recess 17 formed in the tissue-contacting surface 10a, e.g., to minimize twist during the application of the clip 101 to tissue.

As shown in FIG. 11, the second leg portion 420 of the clip 101 includes the recess 417 formed in the force-receiving surface 60 thereof. Second leg portion 420 additionally includes an alignment rib 48 projecting from the tissue-contacting surface 20a thereof. Rib 48 has a height "H₁" and a width "W₄" at the base of the rib 48. Rib 48 shown in FIG. 11 may have the same dimensions as the rib 48 of FIG.5. In embodiments, the width "W₃" at the base of the recess 17 (e.g., shown in FIG. 10) may be substantially the same as the width "W₄" at the base of the rib 48, e.g., to permit tissue exudation during the application of the hemostatic clip 101 to tissue.

A surgical hemostatic clip 102 according to an embodiment of the present disclosure is shown in FIGS. 12 and 13 and includes opposed, first and second leg portions 610 and 620. Surgical hemostatic clip 102 is similar to the hemostatic clip 100 of FIGS. 1 through 3, except for a work-hardened, movement-inhibiting structure 619 defined in the inner surface of the apex 30, and an alignment recess 621 defined in the tissue contacting surface 10a of the first leg portion 610. First and second leg portions 610 and 620 are similar to the first and second leg portions 10 and 20 of the clip 100 shown in FIGS. 1 through 3 and, in the interests of brevity, not all features are discussed.

As best seen in FIG. 13, the work-hardened, movement-inhibiting structure 619 is a recess 617 formed in the inner surface of the apex 30. Recess 617 has a generally C-shaped cross section, and may have a relatively short length in the range of about 0.020 to about 0.090 inches. It may be easier to fabricate a recess of relatively short length disposed at the apex, e.g., as compared to fabrication of the recess 17 of FIGS. 1 through 3. Work-hardened, movement-inhibiting structure 619 formed as a recess 617 defined in the inner surface of the apex 30 may reduce the forces required to apply the clip 102 to ligate blood vessels, nerves or other anatomical structures, as compared to the forces required to apply the clip 100 of FIGS. 1 through 3 or the clip 101 of FIGS. 7 and 8.

Recess 617 formed in the inner surface of the apex 30 shown in FIGS. 12 and 13 may have the same depth and width dimensions as the recess 17 of FIGS. 1 through 3. Recess 617 is formed by cold working the clip 102. It is envisioned and within the scope of the present disclosure that clip 102 embodiments may additionally, or alternatively, include a work-hardened, movement-inhibiting structure formed in the outer surface of the apex 30.

Recess 621 defined in the tissue contacting surface 10a of the first leg portion 610 is configured to receive therein at least a portion of the alignment rib 48 of the second leg portion 620. Rib 48 shown in FIG. 13 may have the same dimensions as the rib 48 of FIG. 5. The shape of the recess 621 and the rib 48 may vary as long as some raised rib member interfits with an appropriately shaped alignment recess.

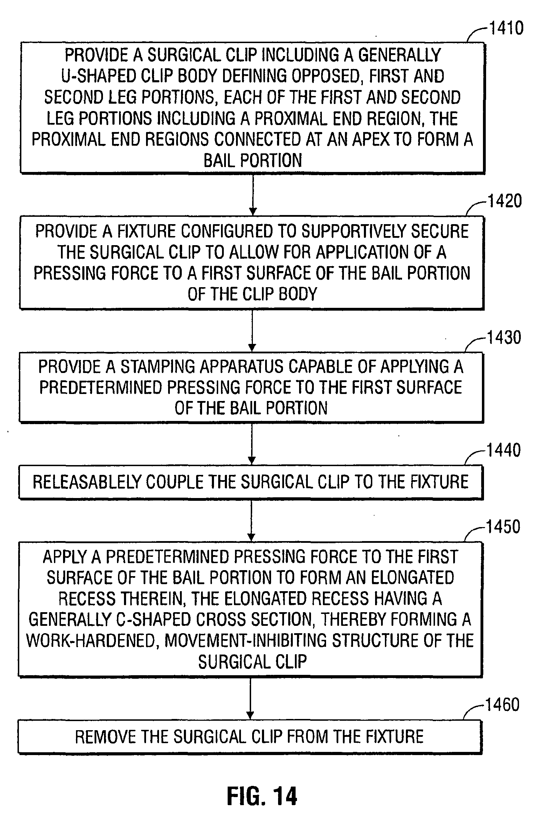

Hereinafter, a method of manufacturing a work-hardened, movement-inhibiting structure of a surgical clip is described with reference to FIG. 14. It is to be understood that the steps of the method provided herein may be performed in combination and in a different order than presented herein without departing from the scope of the disclosure.

In step 1410, a surgical clip (e.g., 100 shown in FIG. 1) is provided including a generally U-shaped clip body defining opposed, first and second leg portions (e.g., 10 and 20 shown in FIG. 1). Each of the first and second leg portions includes a proximal end region (e.g., 13 and 15 shown in FIG. 1). The proximal end regions are connected at an apex (e.g., 30 shown in FIG. 1) to form a bail portion (e.g., 35 shown in FIG. 1).

In step 1420, a fixture is provided. The fixture is configured to supportively secure the surgical clip to allow for application of a pressing force to a first surface of the bail portion of the clip body. In embodiments, the fixture is configured to allow for application of a pressing force to the inner surface (e.g., 31 shown in FIG. 1) of the apex and may be configured to additionally allow for application of a pressing force to one, or both, of the tissue contacting surfaces (e.g., 10a and 20a shown in FIG. 1) of the first and second leg portions.

In step 1430, a stamping apparatus is provided. The stamping apparatus is capable of applying a predetermined pressing force to the first surface of the bail portion. The magnitude of the predetermined pressing force exerted may depend on various factors including temperature and material characteristics of the surgical clip, e.g., density and modulus of elasticity.

In step 1440, the surgical clip is releasablely coupled to the fixture. The surgical clip may be held in place in any suitable fashion, for example, by negative pressure and/or releasable fasteners.

In step 1450, a predetermined pressing force is applied to the first surface of the bail portion to form an elongated recess therein, such that the elongated recess has a generally C-shaped cross section, thereby forming a work-hardened, movement-inhibiting structure of the surgical clip. It is envisioned that a fixture may be designed so as to supportively secure a plurality of surgical clips, in which case a predetermined pressing force may be applied substantially simultaneously to a plurality of surgical clips to form work-hardened, movement-inhibiting structures in accordance with the present disclosure.

In step 1460, the surgical clip including one or more work-hardened, movement-inhibiting structures is removed from the fixture.

The presently disclosed surgical hemostatic clips are designed to exhibit improved closure characteristics, e.g., improved folding characteristics to enhance clip retention on tissue, and may minimize twist during the application of the clip to tissue. The above-described hemostatic surgical clips 100, 101 and 102 include one or more work-hardened, movement-inhibiting structures.

The above-described hemostatic surgical clips may include a work-hardened, movement-inhibiting structure defined in the tissue-contacting surfaces and/or the force-receiving surfaces of the first and second leg portions of the clip. In embodiments, the work-hardened, movement-inhibiting structure may be disposed entirely within the bail portion of the clip. The above-described hemostatic surgical clips 100, 101 and 102 may be fabricated from any surgically suitable material, such as, for example, stainless steel, titanium, tantalum, or other metals or alloys.

Although embodiments have been described in detail with reference to the accompanying drawings for the purpose of illustration and description, it is to be understood that the inventive processes and apparatus are not to be construed as limited thereby. It will be apparent to those of ordinary skill in the art that various modifications to the foregoing embodiments may be made without departing from the scope of the disclosure.
The invention may be described by reference to the following numbered pararaphs:
1. A surgical clip, comprising a clip body defining opposed, first and second leg portions, each of the first and second leg portions including a distal end region and a proximal end region, with the proximal end regions connected at an apex to form a bail portion; and a work-hardened, movement-inhibiting structure defined in the clip body adapted to enhance clip retention on tissue.
2. The surgical clip of paragraph 1, wherein the work-hardened, movement-inhibiting structure is disposed entirely within the bail portion.
3. The surgical clip of paragraph 2, wherein the work-hardened, movement-inhibiting structure is an elongated recess formed by cold working the surgical clip.
4. The surgical clip of paragraph 3, wherein the work-hardened, movement-inhibiting structure is a formed by fixturing the clip and applying a stamping force to an inner surface of the bail portion.
5. The surgical clip of paragraph 1, wherein each of the first and second leg portions includes an inner surface defining a tissue-contacting surface between which tissue is clamped during application of the surgical clip thereto.
6. The surgical clip of paragraph 5, wherein the work-hardened, movement-inhibiting structure includes first and second portions, the first portion disposed in the apex, and the second portion disposed in the tissue-contacting surface of the first leg portion.
7. The surgical clip of paragraph 5, wherein the apex includes an inner surface and the work-hardened, movement-inhibiting structure is an elongated recess formed by applying a stamping force to the inner surface of the apex and the tissue-contacting surface of the first leg portion along a portion of the proximal end region of the first leg portion.
8. The surgical clip of paragraph 7, wherein the elongated recess has a generally C-shaped cross section.
9. The surgical clip of paragraph 5, further comprising a first tissue-gripping structure formed in the tissue-contacting surface of the first leg portion and a second tissue-gripping structure formed in the tissue-contacting surface of the second leg portion.
10. The surgical clip of paragraph 9, wherein the first and second tissue-gripping structure each includes a plurality of multi-faceted, movement-inhibiting structures defining a plurality of recesses disposed in a pattern on the tissue-contacting surfaces at the distal end regions of the first and second leg portions.
11. The surgical clip of paragraph 1, wherein each of the first and second leg portions includes an outer surface defining a force-receiving surface for receiving the compression force of a clip applying apparatus.
12. The surgical clip of paragraph 11, wherein the work-hardened, movement-inhibiting structure is an elongated recess formed in the force-receiving surface.
13. A surgical clip, comprising a clip body defining opposed, first and second leg portions, each of the first and second leg portions including a distal end region and a proximal end region, with the proximal end regions connected at an apex to form a bail portion, each of the first and second leg portions further including an inner surface defining a tissue-contacting surface between which tissue is clamped during application of the surgical clip thereto; and a work-hardened, movement-inhibiting structure defined in at least a portion of the tissue-contacting surface adapted to enhance clip retention on tissue.
14. The surgical clip of paragraph 13, wherein the work-hardened, movement-inhibiting structure is an elongated recess formed by cold working the surgical clip.
15. The surgical clip of paragraph 14, wherein the work-hardened, movement-inhibiting structure is disposed entirely within the bail portion.
16. A method of manufacturing a work-hardened, movement-inhibiting structure of a surgical clip, comprising the steps of providing a surgical clip including a generally U-shaped clip body defining opposed, first and second leg portions, each of the first and second leg portions including a proximal end region, the proximal end regions connected at an apex to form a bail portion; providing a fixture configured to supportively secure the surgical clip to allow for application of a pressing force to a first surface of the bail portion of the clip body; releasablely coupling the surgical clip to the fixture; applying a predetermined pressing force to the first surface of the bail portion to form an elongated recess therein, the elongated recess having a generally C-shaped cross section, thereby forming a work-hardened, movement-inhibiting structure of the surgical clip; and removing the surgical clip from the fixture.
17. The method of manufacturing a surgical clip in accordance with claim 16, further comprising the step of providing a stamping apparatus capable of applying the predetermined pressing force to the first surface of the bail portion.

## Claims

1. A surgical clip, comprising:
a clip body defining opposed, first and second leg portions, each of the first and second leg portions including a distal end region and a proximal end region, with the proximal end regions connected at an apex to form a bail portion; and
a work-hardened, movement-inhibiting structure defined in the clip body adapted to enhance clip retention on tissue.

2. The surgical clip of claim 1, wherein the work-hardened, movement-inhibiting structure is disposed entirely within the bail portion.

3. The surgical clip of claim 2, wherein the work-hardened, movement-inhibiting structure is an elongated recess formed by cold working the surgical clip, preferably wherein the work-hardened, movement-inhibiting structure is a formed by fixturing the clip and applying a stamping force to an inner surface of the bail portion.

4. The surgical clip of any preceding claim, wherein each of the first and second leg portions includes an inner surface defining a tissue-contacting surface between which tissue is clamped during application of the surgical clip thereto.

5. The surgical clip of claim 4, wherein the work-hardened, movement-inhibiting structure includes first and second portions, the first portion disposed in the apex, and the second portion disposed in the tissue-contacting surface of the first leg portion; and/or wherein the apex includes an inner surface and the work-hardened, movement-inhibiting structure is an elongated recess formed by applying a stamping force to the inner surface of the apex and the tissue-contacting surface of the first leg portion along a portion of the proximal end region of the first leg portion.

6. The surgical clip of claim 5, wherein the elongated recess has a generally C-shaped cross section.

7. The surgical clip of claim 5, further comprising a first tissue-gripping structure formed in the tissue-contacting surface of the first leg portion and a second tissue-gripping structure formed in the tissue-contacting surface of the second leg portion.

8. The surgical clip of claim 7, wherein the first and second tissue-gripping structure each includes a plurality of multi-faceted, movement-inhibiting structures defining a plurality of recesses disposed in a pattern on the tissue-contacting surfaces at the distal end regions of the first and second leg portions.

9. The surgical clip of any preceding claim, wherein each of the first and second leg portions includes an outer surface defining a force-receiving surface for receiving the compression force of a clip applying apparatus.

10. The surgical clip of claim 9, wherein the work-hardened, movement-inhibiting structure is an elongated recess formed in the force-receiving surface.

11. A surgical clip, comprising:
a clip body defining opposed, first and second leg portions, each of the first and second leg portions including a distal end region and a proximal end region, with the proximal end regions connected at an apex to form a bail portion, each of the first and second leg portions further including an inner surface defining a tissue-contacting surface between which tissue is clamped during application of the surgical clip thereto; and
a work-hardened, movement-inhibiting structure defined in at least a portion of the tissue-contacting surface adapted to enhance clip retention on tissue.

12. The surgical clip of claim 11, wherein the work-hardened, movement-inhibiting structure is an elongated recess formed by cold working the surgical clip.

13. The surgical clip of claim 11 or claim 12, wherein the work-hardened, movement-inhibiting structure is disposed entirely within the bail portion.

14. A method of manufacturing a work-hardened, movement-inhibiting structure of a surgical clip, comprising the steps of:
providing a surgical clip including a generally U-shaped clip body defining opposed, first and second leg portions, each of the first and second leg portions including a proximal end region, the proximal end regions connected at an apex to form a bail portion;
providing a fixture configured to supportively secure the surgical clip to allow for application of a pressing force to a first surface of the bail portion of the clip body;
releasablely coupling the surgical clip to the fixture;
applying a predetermined pressing force to the first surface of the bail portion to form an elongated recess therein, the elongated recess having a generally C-shaped cross section, thereby forming a work-hardened, movement-inhibiting structure of the surgical clip; and
removing the surgical clip from the fixture.

15. The method of manufacturing a surgical clip in accordance with claim 14, further comprising the step of:
providing a stamping apparatus capable of applying the predetermined pressing force to the first surface of the bail portion.
